# EUROPEAN PATENT APPLICATION

(11) **EP 0 618 297 A1**
(43) Date of publication of application: **05.10.1994**
(21) Application number: 94301735.0
(22) Date of filing: 11.03.1994
(51) Int. Cl.: C12N 15/76, C12N 1/21

(54) **Novel bacterial plasmid shuttle vectors for streptomycetes and escherichia coli**

(30) Priority: 18.03.1993 US 32925
(71) Applicant: PFIZER INC., New York, N.Y. 10017 (US)
(72) Inventor: Denoya, Claudio D., Groton, Connecticut 06340 (US)
(74) Representative: Moore, James William, Dr.

(57) **Abstract**

Four new Escherichia coli - Streptomyces shuttle vectors are described. These shuttle vectors are useful for cloning both in Streptomyces and in E. coli, and for a variety of applications in molecular genetics technology in Streptomyces. Constructs are structurally stable and replicate efficiently in E. coli and in a number of Streptomyces species, allowing transfer of a wide size range of cloned DNA fragments back and forth from one host to another.

## Description

### Background of the Invention

The present invention relates to a collection of novel bacterial shuttle cloning vectors aimed to assist recombinant DNA manipulations in a wide variety of actinomycetes, including the microorganism Streptomyces avermitilis.

Many pharmaceutical products are produced by microorganisms. Microbial participation in the formation of useful products can be divided into three categories based on how much of the key genetic information needed to make the actual chemical structure of the product is present in the microorganism. The first category consists of those products that are natural metabolites, such as antibiotics, all the information for their synthesis being native to the cell. The second category includes those products that are not natural metabolites of microorganisms but the microorganism contributes information for biological steps used in the chemical conversion of chemicals from one form to another. This is termed bioconversion (for example, the bioconversion of steroids). The third category is characterized by the fact that the information that defines the structure of the product molecule is not initially found in the genome of the microorganism. In this case, the information is inserted into the cell. Production of compounds in all three categories has been or will be influenced by recombinant DNA technology. Among those microorganisms with industrial applications, the soil-inhabiting actinomycetes play a central role, and have been recognized as an important source of antibiotics and other biologically important compounds.

Streptomyces, one of the main genera of the order Actinomycetales, have received substantial attention. Members of the genus Streptomyces are gram-positive soil bacteria. Analysis of innumerable isolates from soil samples have yielded more than 90% of the therapeutically useful antibiotics. There is a great deal of industrial experience regarding the growth and handling of actinomycetes over the four decades in which actinomycetes fermentations have been used to produce antibiotics. Additionally, because of interest in improving antibiotic production, genetic studies in actinomycetes are advancing. Actinomycetes are the focus of efforts in developing recombinant DNA cloning techniques in order to isolate antibiotic biosynthetic genes, generate novel derivatives or hybrid compounds, isolate regulatory genes, and investigate the regulatory mechanisms involved in both primary and secondary metabolisms.

While much has been learned about actinomycetes, these microorganisms still present a challenge to molecular biologists. Actinomycetes have many unique structural and metabolical characteristics that differentiate these microorganisms from the rest of the prokaryotes. When actinomycetes grow on a solid medium, they usually exhibit a complex, mycelial fungus-like cycle of morphological differentiation. Mycelial growth and differentiation involve at least three main phases: substrate mycelium, aerial mycelium, and spore formation. In addition, the Streptomyces genome is large, about 10⁴ kb (kilobases), more than twice that of Escherichia coli. The actinomycetes genome is composed of DNA of extremely high guanosine plus cytosine (G+C) content (averaging up to about 73%, some regions more than 90%), close to the upper limit observed in nature. These distinctive characteristics necessitate the development of actinomycetes-specific recombinant DNA techniques.

A basic component in the application of recombinant DNA techniques to the genetic improvement of antibiotic-producing actinomycetes is the development of suitable cloning vectors. A variety of plasmids and phage vectors and transformation systems have been developed. Several vector constructions have been pioneered by the group led by David Hopwood at the John Innes Institute, England. Both, the pIJ101-derived high-copy number plasmid vectors [Kieser, T., Hopwood, D. A., Wright, H. M., and Thompson, C. J. Mol. Gen. Genet, 185, 223-238, 1982], and the SCP2*-derived low copy number plasmid vectors [Lydiate, D. J., Malpartida, F., and Hopwood, D. A., Gene, 35, 223-235, 1985], have been successfully used in cloning actinomycetes DNA. SCP2* is a fertility factor originally found in Streptomyces coelicolor with a molecular size of 31.4 kb and exists in one to two copies per chromosome [Bibb, M. J. and Hopwood, D. A. J. Gen Microbiol., 126, 427-442, 1981]. SCP2* has a broad host range. Two widely used actinomycetes vectors are: the low copy number vector plJ922 [Lydiate et al, Gene, 35, 223-235, 1985], a derivative of the SCP2* replicon originally isolated from S. coelicolor, and the high-copy number vector pIJ702 [Katz, E, Thompson, C. J., and Hopwood, D. A., J. Gen. Microbiol. 129, 2703-2714, 1983]], a derivative of plasmid pIJ101 originally isolated from S. lividans. Both vectors carry the thiostrepton-resistant (tsr) marker from Streptomyces azureus, which confers resistance to the antibiotic thiostrepton by a specific ribosomal RNA methylase. Depending on the specific project, a decision is made on the copy number of the vector used. Vectors with low or single copy number per genome are preferred for most physiological studies, where the ability to stably maintain large DNA fragments (up to 40 kb), and the absence of possible gene dosage effects, are preferred. Sometimes the analysis of a cloned segment of DNA by complementation of specific mutations is hampered by the instability of transformants obtained with many of the available cloning vectors. Those with a high copy number, such as plJ702 (40 to 300 copies per chromosome), are valuable for cloning and easiness of recovery of cloned DNA fragments. plJ702 also has a broad range of suitable actinomycetes hosts.

Genetic engineering work in actinomycetes is time-consuming compared to E. coli systems. Bifunctional plasmid vectors, also known as shuttle vectors, are able to replicate in Streptomyces and one or more alternative hosts (e.g., E. coli) greatly facilitating cloning, restriction mapping, DNA sequencing, site-directed mutagenesis, and functional analysis of actinomycetes genes. Bifunctional Streptomyces-E. coli shuttle vectors, containing both an E. coli and an actinomycetes replicon, as well as selective markers specific for each host, are capable of stable maintenance in both E. coli and Streptomyces. Shuttle vectors can be transformed between both alternative hosts, greatly facilitating gene transfer and molecular genetics studies of actinomycetes genes. One or more genes cloned from an actinomycetes host into a shuttle vector, can then be transferred to an E. coli host. Once the gene(s) under study has been analyzed and manipulated in the E. coli host, it can then be transferred back to its original actinomycetes host.

The present invention discloses the construction of four novel bifunctional plasmid shuttle vectors for actinomycetes, preferably Streptomycetes and Escherichia coli. The novel vectors have been designed to facilitate a wide number of recombinant DNA applications in actinomycetes and related microorganisms. Three of the vectors carry a temperature-sensitive origin of replication that controls for a moderate-to-high copy number in Streptomyces. The temperature sensitivity of this replicon allows its application in Streptomyces gene replacement and mutant design experiments. In addition, one of these shuttle vectors contains both SP6 and T7 RNA polymerase promoters flanking the multiple cloning region, allowing the synthesis of cRNA transcripts useful for hybridization studies. Additionally, the cloning region is located within the alpha-peptide coding region of the beta-galactosidase gene, allowing recombinant clones to be identified by color screening in Escherichia coli. The fourth shuttle vector described here, carries the SCP2* Streptomyces origin of replication that controls for a low copy number in Streptomyces, which constitutes an advantageous characteristic for a vector utilized in cloning and complementation studies in many Streptomyces species.

Technical terms used throughout this application are well known to those skilled in the art of molecular genetics. Definition of those terms are found in many textbooks dedicated to the molecular biology field, such as "Genes", Second Edition, by Dr. Benjamin Lewin, 1985, John Wiley & Sons, Inc. New York. Terms frequently utilized in this invention are defined below:

Antibiotic: A chemical agent that inhibits growth of bacterial cells. Used to select recombinant bacterial cells.

Antibiotic Resistance Gene: DNA sequence that conveys resistance to antibiotic when introduced into a host cell that is naturally sensitive to that particular antibiotic. Also know as antibiotic marker.

cRNA: Single-stranded RNA complementary to a DNA, synthesized from it by in vitro transcription.

Clone: Large number of cells or molecules identical with a single ancestor.

Cloning Vector: Any plasmid into which a foreign DNA may be inserted to be cloned. It carries foreign DNA into a host bacterial cell upon transformation.

DNA Ligation: Is the formation of a chemical bond to link two fragments of DNA.

Hybridization: Technique used to identify bacterial colonies carrying chimeric vectors whose inserted DNA is similar with some particular sequence.

kb: Abbreviation for 1,000 base pairs of DNA or RNA.

Origin of Replication: DNA sequence at which replication of the plasmid vector is initiated.

Plasmid: Autonomous self-replicating extrachromosomal circular DNA.

Plasmid Copy Number: Number of plasmid molecules maintained in bacteria for every host chromosome.

Promoter: (Also known as RNA Polymerase promoter) Region of DNA important to define the initiation of transcription of a particular DNA sequence into RNA.

Replicon: Unit of genome in which DNA is replicated; contains an origin of replication.

Restriction Enzyme: Enzymatic protein that recognizes specific short sequence of DNA and cleaves it.

Shuttle Vector: Bifunctional cloning vector able to replicate in one or more alternative hosts (e.g., E. coli and Streptomyces).

Temperature-Sensitive Mutation: Creates a gene product that is functional at low temperature but inactive at higher temperature.

Transcription: Synthesis of RNA on a DNA template.

Transformation of Bacterial Cells: Describes the acquisition of new genetic markers by incorporation of added DNA.

### Summary of the Invention

The present invention relates to recombinant DNA shuttle vectors that control for a moderate to high copy number in streptomycetes cells comprising:
(a) the origin of replication-containing SacI restriction fragment approximately 5.7 kilobases in size of plasmid pMT660;
(b) a DNA segment containing at least one antibiotic marker that confers resistance when transformed into a sensitive Streptomyces host cell;
(c) the origin of replication-containing SacI restriction fragment approximately 2.7 kilobases in size of plasmid pGEM-3Z;
(d) a DNA segment containing at least one antibiotic marker that confers resistance when transformed into a sensitive E. coli host cell;
(e) four convenient cloning sites: BgIII, EcoRI, HindIII, and XbaI; and
(f) said vector capable of replicating efficiently in both a wide variety of streptomycetes and E. coli hosts.

The present invention also relates to recombinant DNA shuttle vectors that control for a moderate to high copy number in streptomycetes cells comprising:
(a) the origin of replication-containing SacI restriction fragment approximately 5.7 kilobases in size of plasmid pMT660;
(b) a deletion of PstI restriction fragment approximately 0.8 kilobases in size;
(c) a DNA segment containing at least one antibiotic marker that confers resistance when transformed into a sensitive Streptomyces host cell;
(d) the origin of replication-containing SacI restriction fragment approximately 2.7 kilobases in size of plasmid pGEM-3Z;
(e) a DNA segment containing at least one antibiotic marker that confers resistance when transformed into a sensitive E. coli host cell;
(f) five convenient cloning sites: EcoRI, HindIII, SacI, SphI, and PstI;
(g) said vector capable of replicating efficiently in both a wide variety of streptomycetes and E. coli hosts.

The present invention also relates to recombinant DNA shuttle vectors that control for a moderate to high copy number comprising:
(a) the blunt-ended origin of replication-containing SacI restriction fragment approximately 5.7 kilobases in size of plasmid pMT660;
(b) a DNA segment containing at least one antibiotic marker that confers resistance when transformed into a sensitive Streptomyces host cell;
(c) the blunt-ended origin of replication-containing NdeI restriction fragment approximately 2.7 kilobases in size of plasmid pGEM-3Z;
(d) a DNA segment containing at least one antibiotic marker that confers resistance when transformed into a sensitive E. coli host cell;
(e) six convenient cloning sites: BgIII, EcoRI, HindIII, SacI, SspI, and XbaI;
(f) four of the cloning sites (EcoRI, HindIII, SacI, and XbaI) located within the alpha-peptide coding region of the beta-galactosidase gene, allowing recombinant clones to be identified by color screening in E. coli;
(g) SP6 and T7 RNA polymerase promoters flanking the cloning sites (e) allowing the synthesis of cRNA transcripts useful for hybridization studies; and
(h) said vector capable of replicating efficiently in both a wide variety of streptomycetes and E. coli hosts.

The present invention also relates to a recombinant DNA shuttle vector that controls for a low copy number in streptomycetes cells comprising:
(a) the origin of replication-containing BamHI/EcoRI restriction fragment approximately 23.4 kilobases in size of plasmid plJ922;
(b) a DNA segment containing at least one antibiotic marker that confers resistance when transformed into a sensitive Streptomyces host cell;
(c) the origin of replication-containing BamHI/EcoRI restriction fragment approximately 3.2 kilobases in size of plasmid pGEM-3Zf(+);
(d) a DNA segment containing at least one antibiotic marker that confers resistance when transformed into a sensitive E. coli host cell,
(e) four convenient cloning sites: BamHI, EcoRI, HindIII, and XhoI; and
(f) said vector capable of replicating efficiently in both a wide variety of streptomycetes and E. coli hosts.

Thus four new shuttle vectors functional in both E. coli and Streptomyces are preferred embodiments of this invention described herein.

Three of these shuttle vectors (pCD262, pCD385, and pCD500) carry a Streptomyces temperature-sensitive origin of replication that controls for a moderate-to-high copy number in Streptomyces. The temperature-sensitive origin of replication is derived from plasmid pMT660, a derivative of plasmid plJ702 obtained after in vitro mutagenesis using hydroxylamine [Birch, A. W., and Cullum, J, J. Gen. Microbiol., 131, 1299-1303, 1985). The temperature sensitivity of this replicon allows its application in Streptomyces gene replacement and mutant design experiments. In addition, shuttle vector pCD385 contains both SP6 and T7 RNA polymerase promoters flanking the multiple cloning region, allowing the synthesis of cRNA transcripts useful for hybridization studies. Additionally, the cloning region is located within the alpha-peptide coding region of the beta-galactosidase gene, allowing recombinant clones to be identified by color screening in Escherichia coli.

The fourth shuttle vector pCD396 carries the SCP2* Streptomyces origin of replication that controls for a low copy number in Streptomyces, which constitutes an advantageous characteristic for a vector utilized in cloning and complementation studies in many Streptomyces species.

### Brief Description of the Figures

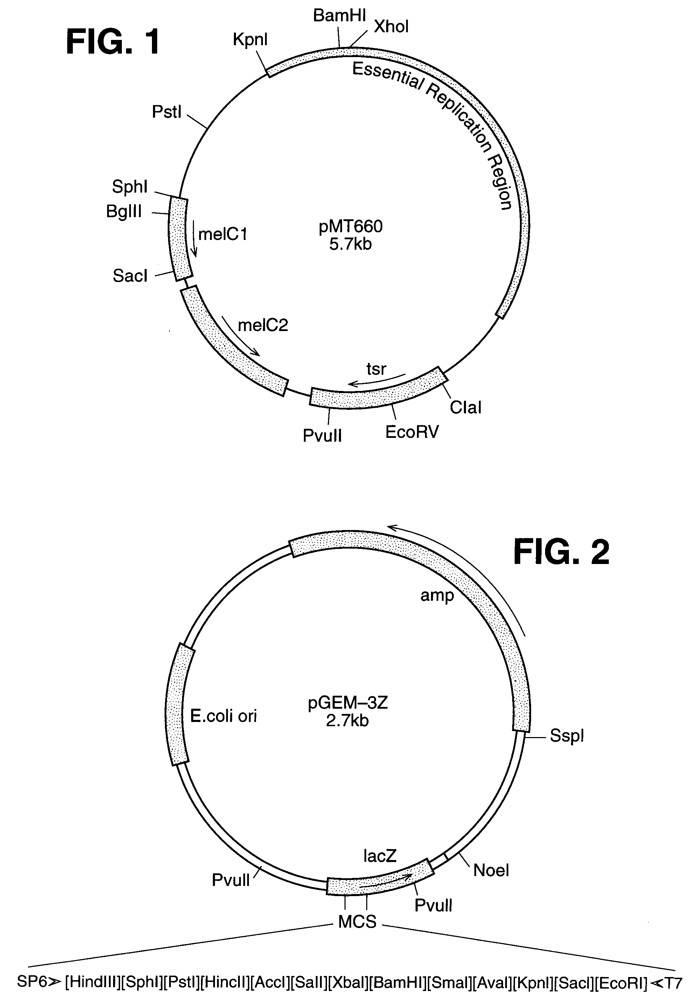

- Fig. 1: shows a restriction map of plasmid vector pMT660.
- Fig. 2: shows a restriction map of plasmid vector pGEM-3Z.

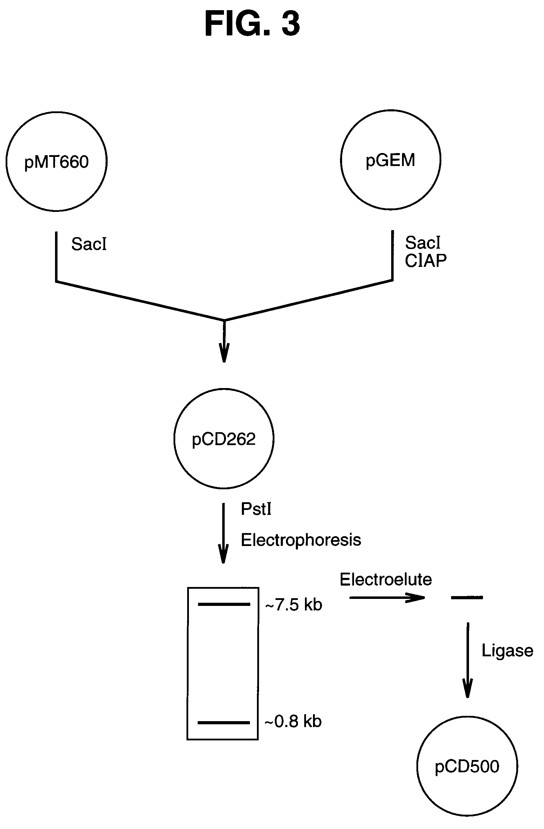

- Fig. 3: shows the construction of plasmid shuttle vectors pCD262 and pCD500. pCD262 is a hybrid between the Streptomyces vector pMT660 and the E. coli vector pGEM-3Z linked at their unique SacI restriction sites. pCD500 is a derivative of pCD262 constructed by deleting 0.8kb PstI fragment present in pCD262.
- Fig. 4: shows a restriction map of plasmid shuttle vector pCD262.
- Fig. 5: shows a restriction map of plasmid shuttle vector pCD500.

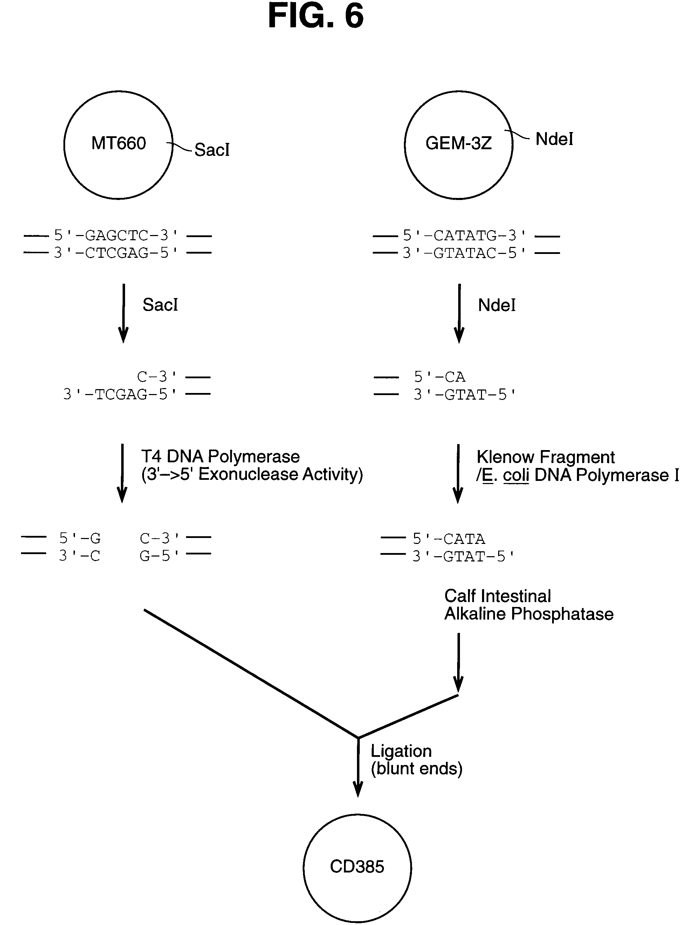

- Fig. 6: shows the construction of plasmid shuttle vector pCD385 which is a hybrid between the Streptomyces vector pMT660 and the E. coli vector pGEM-3Z linked at their unique SacI and NdeI restriction sites, respectively. This construction is aimed to preserve the multiple cloning region present in pGEM-3Z intact.
- Fig. 7: shows a restriction map of plasmid shuttle vector pCD385.
- Fig. 8: shows a restriction map of plasmid shuttle vector pCD396.

### Detailed Description of the Invention

The present invention discloses the construction of four stable bifunctional plasmid shuttle vectors for Streptomyces and Escherichia coli. The novel vectors have been designed to facilitate a wide number of recombinant DNA applications in actinomycetes and related microorganisms. Three of the vectors carry a temperature-sensitive derivative of the pIJ101 origin of replication that controls for a moderate-to-high copy number in Streptomyces. The temperature sensitivity of this replicon allows its application in Streptomyces gene replacement and mutant design experiments. The fourth shuttle vector described here, carries the SCP2* Streptomyces origin of replication that controls for a low copy number in Streptomyces, which constitutes an advantageous characteristic for a vector utilized in cloning and complementation studies in many Streptomyces species.

These shuttle vectors are useful for cloning both in Streptomyces and in Escherichia coli, and for a variety of applications in molecular genetics technology in Streptomyces. Constructs are structurally stable and replicate in a number of Streptomyces species, allowing transfer of a wide size range of cloned DNA fragments back and forth from one host to another. These vectors have the widely used ColE1 origin of replication [Hershfield, V, Boyer, H. W., Yanofsky C., Lovett, M. A., and D. R. Helinski, Proc. Natl. Acad. Sci., 71:3455, 1974], and efficiently replicate in all Escherichia coli strains commonly used in recombinant DNA experiments. These vectors carry both the tsr gene, which confers resistance to the antibiotic thiostrepton upon transformation into actinomycetes cells, and the amp gene, which confers resistance to the antibiotic ampicillin upon transformation into E. coli cells. Three of the shuttle vectors described in the present invention carry a Streptomyces temperature-sensitive origin of replication that controls for a moderate-to-high copy number in Streptomyces. The temperature sensitivity of this replicon plus its poor survival under environmental stresses, allow its application in Streptomyces gene replacement and mutant design experiments. One of these shuttles contains SP6 and T7 RNA polymerase promoters flanking the multiple cloning region, allowing the synthesis of cRNA transcripts useful for hybridization studies. Additionally, the cloning region is located within the alpha-peptide coding region of the beta-galactosidase gene, allowing recombinant clones to be identified by color screening in E. coli. The fourth shuttle vector described herein, carries a Streptomyces origin of replication that controls for a low copy number in Streptomyces, which constitutes an advantageous characteristic for a vector utilized in cloning and complementation studies in many Streptomyces species.

The plasmid shuttle vector pCD262 comprises:
a. an approximately 5.7 kb SacI pMT660 moiety containing: a temperature-sensitive plJ702-derived actinomycetes origin of replication and a DNA sequence that conveys resistance to thiostrepton when transformed into a sensitive actinomycetes host cell; and
b. an approximately 2.7 kb SacI pGEM-3Z (Promega Corporation Madison, WI) moiety containing: a ColE1 E. coli origin of replication and a DNA sequence that conveys resistance to the antibiotic ampicillin when transformed into a sensitive E. coli host cell.

As a result of this construction, plasmid pCD262 has the following useful characteristics:
a. It has a medium size (8.4 kb) allowing cloning of a wide size range of DNA fragments;
b. It is structurally stable and replicates efficiently in both a wide variety of actinomycetes and E. coli hosts;
c. It has four convenient cloning sites: BgIII, EcoRI, HindIII, and XbaI; and
d. It has a temperature-sensitive actinomycetes replicon which allows its application in Streptomyces gene replacement and mutant design experiments.

The plasmid shuttle vector pCD500 was constructed by deleting an approximately 0.8 kb Pstl fragment present in plasmid pCD262. As a result of this deletion, the following additional useful characteristics were obtained:
a. It has a smaller size (7.6 kb); and
b. It has five convenient cloning sites: EcoRI, HindIII, SacI, SphI, and PstI.

The plasmid shuttle vector pCD385 comprises:
a. an approximately 5.7 kb SacI pMT660 moiety that was blunt-ended by the action of the 3' → 5' exonuclease activity present in the bacteriophage T4 DNA polymerase. This moiety contains: a temperature-sensitive plJ702-derived actinomycetes origin of replication and a DNA sequence that conveys resistance to thiostrepton when transformed into a sensitive actinomycetes host cell; and
b. an approximately 2.7 kb NdeI pGEM-3Z moiety that was blunt-ended by a filling reaction catalyzed by the Klenow fragment of the E. coli DNA polymerase I.

This moiety contains a DNA segment derived from the lac operon of Escherichia coli that codes for the amino-terminal fragment of beta-galactosidase [Yanisch-Perron,C., Vieira, J., and J. Messing, Gene 33, 103, 1985]. This fragment, whose synthesis can be induced by isopropylthio-beta-D-galactoside (IPTG), is capable of intra-allelic (alpha) complementation with a defective form of beta-galactosidase encoded by the host. E. coli cells exposed to the inducer IPTG synthesize both fragments of the enzyme and form blue colonies when plated on media containing the chromogenic substrate 5-bromo-4-chloro-3-indolyl-beta-D-galactoside (X-gal). Insertion of foreign DNA into the polycloning site of the plasmid inactivates the amino-terminal fragment of the beta-galactosidase and abolishes alpha-complementation. Bacteria carrying recombinant plasmids therefore give rise to white colonies. In addition, both bacteriophage SP6 and T7 RNA polymerase promoters are located flanking the multiple cloning region, allowing the synthesis of cRNA transcripts useful for hybridization studies. The pGEM-3Z moiety additionally contains a ColE1 E. coli origin of replication, and a DNA sequence that conveys resistance to the antibiotic ampicillin when transformed into a sensitive E. coli host cell.

As a result of this construction, plasmid pCD385 has the following useful characteristics:
a. It has a medium size (8.4 kb) allowing cloning of a wide size range of DNA fragments;
b. It is structurally stable and replicates efficiently in both a wide variety of actinomycetes and E. coli hosts;
c. It has six convenient cloning sites: BgIII, EcoRI, HindIII, SacI, SspI, and XbaI;
d. Four of the cloning sites (EcoRI, HindIII, SacI, and XbaI) are located within the alpha-peptide coding region of the beta-galactosidase gene, allowing recombinant clones to be identified by color screening in E. coli;
e. It has SP6 and T7 RNA polymerase promoters flanking the restriction sites mentioned above, allowing the synthesis of cRNA transcripts useful for hybridization studies; and
f. It has a temperature-sensitive actinomycetes replicon which allows its application in Streptomyces gene replacement and mutant design experiments.

The plasmid shuttle vector pCD396 comprises:
a. an approximately 23.4 kb BamHI/EcoRI pIJ922 moiety containing: a SCP2* actinomycetes origin of replication and a DNA sequence that conveys resistance to thiostrepton when transformed into a sensitive actinomycetes host cell; and
b. an approximately 3.2 kb BamHI/EcoRI pGEM-3Zf(+) moiety containing: a ColE1 E. coli origin of replication and a DNA sequence that conveys resistance to the antibiotic ampicillin when transformed into a sensitive E. coli host cell.

As a result of this construction, plasmid pCD396 has the following useful characteristics:
a. It is structurally stable and replicates efficiently in both a wide variety of streptomycetes and E. coli hosts;
b. It has four convenient cloning sites: BamHI, EcoRI, HindIII, and XhoI;
c. It has a XhoI cloning site, which allows the application of a simplified genomic cloning strategy, as described by Zabarovsky and Allikmets, Gene, 42:119, 1986; and
d. It has a actinomycetes origin of replication that controls for a low copy number in Streptomyces which constitutes an advantageous characteristic for complementation studies.

The following have been deposited under the terms of the Budapest Treaty in the American Type Culture Collection, Rockville, Maryland, a recognized depository affording permanence of the deposits and ready accessibility thereto by the public if a patent is granted on this application. The deposits are available during pendency of this application to one determined by the Commissioner of the United States Patent and Trademark Office to be entitled thereto under 37 CFR 1.14 and 35 USC 122, and in accordance with foreign patent laws, in countries wherein counterparts of this application, or its progeny, are filed. All restrictions on the availability to the public of the microorganisms deposited will be irrevocably removed upon granting of the patent.

| ATCC DEPOSITS IN ACCORDANCE WITH THE PRESENT INVENTION | |
|---|---|
| Identification Reference by Depositor Pfizer, Inc. | ATCC Designation |
| Escherichia coli, FD 29390 | 69432 |
| Escherichia coli, FD 29391 | 69433 |
| Escherichia coli, FD 29401 | 69436 |
| Escherichia coli, FD 29393 | 69435 |
| S. lividans TK64, FD 29405 | 69442 |
| S. lividans TK64, FD 29418 | 69444 |
| S. lividans TK64, FD 29404 | 69441 |
| S. lividans TK64, FD 29406 | 69443 |

The following are detailed Examples of the construction of the novel plasmid shuttle vectors, pCD262, pCD500, pCD385, and pCD396 which are also illustrated in the accompanying Figures.

### EXAMPLE 1

### Large Scale Preparation of Plasmid Vectors from Streptomyces Strains

Streptomyces lividans strain TK64 containing plasmid vector pIJ922 [Lydiate et al, Gene, 35, 223-235, 1985] and S. lividans TK64 containing plasmid vector pMT660 [Birch, A. W., and Cullum, J, J. Gen. Microbiol., 131, 1299-1303, 1985] were kindly supplied by Dr. D. A. Hopwood (The John Innes Institute, Norwich, England). S. lividans strain TK64 has been very well analyzed and is widely used in actinomycetes research. A detailed description of this strain can be found in the textbook "Genetic Manipulation of Streptomyces, A Laboratory Manual", The John Innes Foundation, Norwich, U.K., 1985, in which, additionally, other procedures for handling actinomycetes cultures, and basic molecular biology protocols applied to the study of actinomycetes cells are also described in detail. S. lividans strains were grown on R2YE agar plates. R2YE medium (Thompson, C.J., Ward, J.M., and D. A. Hopwood, 1980, Nature, 286:525-527) was prepared by dissolving 10.3 g sucrose, 0.025 g K₂SO₄, 1.012 g MgCl₂.6H₂O, 1 g glucose, 0.01 g Casamino acids, and 2.2 g agar in 80 ml distilled water and autoclaved for 40 minutes at 121°C. Then, the following compounds were sequentially added as filter sterilized solution in water: 0.2 ml trace element solution (per liter: 40 mg ZnCl₂, 200 mg FeCl₃.6H₂O, 10 mg CuCl₂.2H₂O, 10 mg MnCl₂.4H₂O, 10 mg Na₂B₄O₇.10H₂O, and 10 mg(NH₄)₆Mo₇O₂₄.4H₂O), 1 ml 0.5% KH₂PO₄, 8.02 ml 3.68% CaCl₂.2H₂O, 1.5 ml 20% L-proline, 10 ml TES buffer (0.25 M Tris-HCl, pH 7.2; 0.05 M sodium EDTA; 0.5 M NaCI), 0.5 ml 1 N NaOH, and 5 ml 10% yeast extract. Plasmid DNA was prepared from cultures grown in liquid YEME medium (Bibb, M.J., Freeman, R. F., and D. A. Hopwood, 1977, Mol. Gen. Genetics, 154:155-166). YEME medium contained per liter: 3 g yeast extract, 5 g bacto-peptone, 3 g malt extract, 340 g sucrose. After autoclaving for 40 minutes at 121°C, 2 ml of 2.5 M MgCl₂.6H₂O were added. Cultures were grown for 40-48 h at 30°C. Mycelium from 500 ml culture of actinomycetes cells were harvested by centrifugation, and resuspended to a final volume of 50 ml with lysozyme solution (2 mg/ml lysozyme in 0.3 M sucrose, 0.025 M Tris-HCl (pH 8.0), and 0.025 M EDTA). After this step, plasmids were prepared following an alkaline lysis procedure, essentially as described in the manual: "Genetic Manipulation of Streptomyces, A Laboratory Manual", The John Innes Foundation, Norwich, 1985. DNA pellets were redissolved in 500 µl 10 mM Tris-HCl, pH 8.0, 1 mM EDTA, and further purified by cesium chloride-ethidium bromide gradient ultracentrifugation essentially as described by Maniatis et al, Molecular Cloning - a laboratory manual, Cold Spring Harbor Laboratory, 1989. The isolated plasmid DNA is finally dissolved in DNA buffer (10 mM Tris-HCl, 4 mM NaCI, 0.1 mM EDTA; pH 7.5) to produce a concentration of approximately 1 µg of plasmid DNA per 10 µl of buffer.

### EXAMPLE 2

### Large Scale Preparation of Plasmid Vectors from E. coli strains

Ampicillin-resistant transformants of E. coli strain carrying replicating vectors were obtained by transformation of competent E. coli K12 DH5-α cells with the plasmid of interest following standard protocols as described by Maniatis et al, Molecular Cloning - a laboratory manual, Cold Spring Harbor Laboratory, 1989. Competent E. coli strain DH5-alpha cells (purchased from Li fe Technologies, Inc., Gaithersburg, MD) were transformed with purified plasmid DNA by the method recommended by the suppliers. A single bacterial colony of a particular E. coli transformant was inoculated into sterile LB medium which contains, per liter aqueous solution, 10 g bacto tryptone, 5 g bacto-yeast extract, and 10 g NaCI (pH 7.0) with 50 mg of ampicillin following standard microbiological procedures. The culture was incubated at 35°C overnight.

The following morning, the bacterial cells were harvested by centrifugation at 10,000 rpm for 5 min at 4°C. Vector DNA was isolated from fresh harvested Escherichia coli cells using a modification of the method of Birnboim and Doly (Nucleic Acids Res., 1979, 7: 1513), as described by Denoya et al. (Microbios Lett., 1985, 29:87). DNA pellets were redissolved in 500 µl 10 mM Tris-HCl, pH 8.0, 1 mM EDTA, and further purified by cesium chloride-ethidium bromide gradient ultracentrifugation essentially as described by Maniatis et al, Molecular Cloning - a laboratory manual, Cold Spring Harbor Laboratory, 1989. The isolated plasmid DNA is finally dissolved in DNA buffer (10 mM Tris-HCl, 4 mM NaCI, 0.1 mM EDTA; pH 7.5) to produce a concentration of approximately 1 µg of plasmid DNA per 10 µl of buffer.

### EXAMPLE 3

### Construction of Shuttle Vector pCD262

Plasmid shuttle vector pCD262 is a hybrid between the Streptomyces vector pMT660 and the E. coli vector pGEM-3Z linked at their unique Sacl restriction sites.

### A. SacI Digestion of Plasmid pMT660

pMT660 is a 5.7 kb Streptomyces plasmid containing a temperature-sensitive origin of replication that controls for a moderate-to-high copy number in Streptomyces. The temperature-sensitivity characteristic of this origin of replication was obtained after hydroxylamine in vitro mutagenesis of its parental plasmid plJ702 [Birch, A. W., and Cullum, J, J. Gen. Microbiol., 131, 1299-1303, 1985]. Purified plasmid pMT660 was prepared from a S. lividans transformant as described in Example 1. The techniques used for cleaving plasmid DNA at specific sites in vitro, ligating previously cleaved DNA, and the designations of the particular enzymes used are well known to those skilled in molecular biology and are described, for example, in the laboratory manual " Molecular Cloning" by Maniatis et al (Cold Spring Harbor Laboratory, 1989). Following this, approximately 3 µg of the plasmid pMT660 in 26 µl of DNA buffer were incubated with 10 units of the restriction enzyme SacI (all restriction enzymes were purchased from Boehringer Mannheim Biochemicals) in the assay buffer specified by the supplier, at 37° C for 4 hours, in a total reaction volume of 30 µl. Following restriction with SacI, the DNA was extracted twice with an equal volume of phenol-chloroform, twice with an equal volume of ether, and finally the DNA was precipitated by adding 2 volumes of absolute ethanol. Precipitated DNA was recovered by centrifugation at 10,000 x G for 10 minutes and dried under vacuum. The SacI-treated DNA was then dissolved in 12 µl of DNA buffer.

### B. Sacl Digestion and Dephosphorylation of Plasmid pGEM-3Z

pGEM-3Z is a 2.7 kb E. coli vector purchased from Promega Corporation, Madison, WI. Purified plasmid pGEM-3Z was prepared from an E. coli transformant as described in Example 1. Following this approximately 1 µg of the plasmid pGEM-3Z in 1 µl of DNA buffer was incubated with 10 units of the restriction enzyme SacI (Boehringer Mannheim Biochemicals) in the assay buffer specified by the supplier, at 37°C for 3.5 h, in a total reaction volume of 20 µl. Then, the SacI-linearized pGEM-3Z was dephosphorylated using calf intestine alkaline phosphatase (CIAP) (purchased from Promega Corp., Madison, WI) following the instructions obtained from the supplier. The reaction mixture was incubated for 30 minutes at 37°C, and the DNA was then extracted consecutively with phenol/chloroform and ether, ethanol precipitated, and pelleted by centrifugation. The final pellet was redissolved in 10 µl of DNA buffer.

### C. Ligation to Produce pCD262

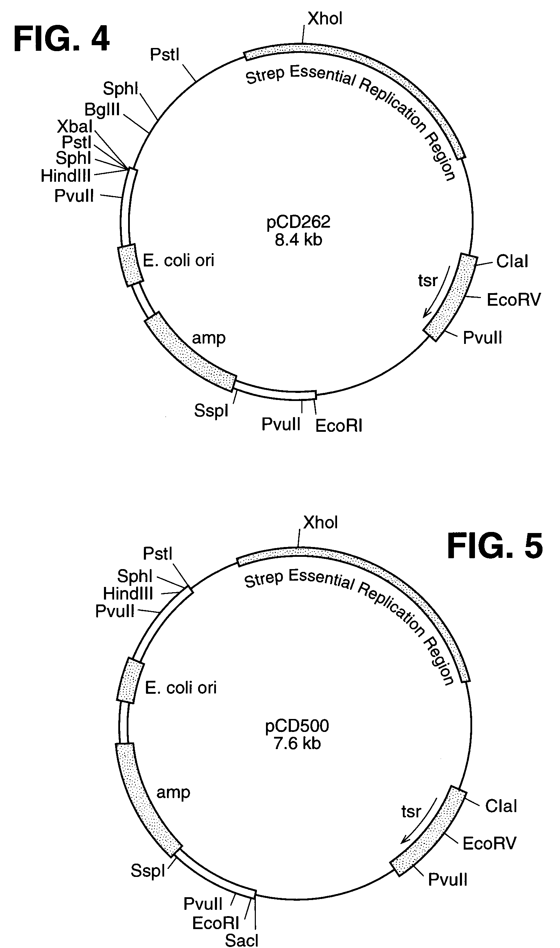

About 10 µl of the 5.7 kb SacI-linearized pMT660 (see Example 3A), and about 2 µl of the 2.7 kb SacI-linearized, CIAP-dephosphorylated pGEM-3Z were incubated overnight with 1 unit of ligase (New England BioLabs, Inc., Beverly, MA) under the conditions specified by the supplier at 15°C in a total reaction volume of 20 µl. Next morning, the ligation mixture was used to transform competent E. coli strain DH5-alpha (purchased from Life Technologies, Inc., Gaithersburg, MD) by the method recommended by the suppliers. Many ampicillin-resistant transformants were recovered. These colonies should contain the plasmid pCD262, which includes a unique restriction site map. This was confirmed by selecting one colony, designated as strain CD262, isolating plasmid DNA from this strain (by the method previously described in Example 2), and constructing a restriction map. A restriction map of pCD262 is presented in Figure 4 of the accompanying drawings.

### EXAMPLE 4

### Construction of Shuttle Vector pCD500

### A. Pstl Digestion of Shuttle Vector pCD262 and Isolation of the approximately 7.6 kb Restriction Fragment

About 3 µg of purified plasmid pCD262 and 10 units of PstI restriction enzyme were incubated in the assay buffer specified by the supplier, at 37°C for 2 hours, in a total reaction volume of 30 µl. The sample was then subjected to electrophoresis in a horizontal 0.8% agarose gel in 1X TBE buffer (90 mM Tris-HCl, pH 8.5, 90 mM Boric acid, 2.5 mM EDTA) for 1.5 hours at 100 V as described by Maniatis et al. The separated DNA fragments were located in the gel by staining with ethidium bromide and visualizing fluorescent bands with a 365 nm ultraviolet light. The 7.6 kb DNA band was sliced with a razor blade and the DNA recovered from the agarose gel by electroelution for 50 min at 80 V into a V-shaped well filled with 7.5 M ammonium acetate using a unidirectional electroelutor (International Biotechnology Inc., New Haven, CT). The DNA was then precipitated with ethanol, pelleted and finally redissolved in 22 µl of DNA buffer.

### B. Ligation to Produce pCD500

About 12 µl of the electroeluted 7.6 kb PstI pCD262 DNA fragment (see Example 4A) were incubated overnight with 1 unit of ligase (New England BioLabs, Inc., Beverly, MA) under the conditions specified by the supplier at 16°C in a total reaction volume of 20 µl. Next morning, the ligation mixture was used to transform competent E. coli strain DH5-alpha as previously discussed. The selected transformant was designated as strain CD500. The identified transformant was then used for subsequent production and isolation of shuttle vector pCD500. A restriction map of shuttle vector pCD500 is presented in Figure 5.

### EXAMPLE 5

### Construction of Shuttle Vector pCD385

Plasmid shuttle vector pCD385 is a hybrid between the Streptomyces vector pMT660 and the E. coli vector pGEM-3Z linked at their unique SacI and NdeI restriction sites, respectively. This construction is designed to preserve the multiple cloning site region present in pGEM-3Z intact.

### A. SacI Digestion of pMT660 and Repair of the 3' Overhanging Ends to Generate Blunt Ends

Accordingly, approximately 3 µg of pMT660 were linearized with the restriction enzyme SacI following procedures essentially similar to those described in Example 3A. Then, the DNA sample was extracted twice with an equal volume of phenol-chloroform, twice with an equal volume of ether, and finally the DNA was precipitated by adding 2 volumes of absolute ethanol. Precipitated DNA was recovered by centrifugation at 10,000 x G for 10 minutes and dried under vacuum. The SacI-treated DNA was finally dissolved in 20 µl of DNA buffer. About 18 µl of SacI-treated pMT660 were then treated with 10 units of bacteriophage T4 DNA polymerase (purchased from BRL/Gibco) in a reaction mixture containing 31 mM Tris-Acetate buffer, pH 7.9, 10 mM magnesium acetate, 66 mM sodium acetate, 0.1 mM of each deoxynucleotide, and 10 mM DTT, for 15 minutes at 11°C. Then 4 µl of 0.5 M EDTA, pH 8.0, were added to inactivate the enzyme. Under these conditions, the strong 3' -> 5' exonuclease activity present in the T4 DNA polymerase will stop degrading the 3' protruding ends when it reaches the duplex region. The DNA was then extracted, precipitated, and resuspended in 20 µl of DNA buffer as previously described.

### B. NdeI Digestion of pGEM-3Z, Repair of the 5' Overhanging Ends to Generate Blunt Ends. and Dephosphorylation

Accordingly, approximately 1.5 µg of the plasmid pGEM-3Z in 13 µl of DNA buffer was incubated with 10 units of the restriction enzyme NdeI (Boehringer Mannheim Biochemicals) in the assay buffer specified by the supplier, at 37°C for 2 hours, in a total reaction volume of 40 µl. Then, 4 µl of 0.5 mM dNTP (0.5 mM each of the 4 dNTPs), and 6 units of the Klenow fragment of Escherichia coli DNA polymerase I (Boehringer Mannheim Biochemicals) were added and the mixture incubated further at 30°C for 15 minutes The repairing reaction was stopped by adding 2 µl of 0.5 M EDTA, pH 8.0, and the DNA was then extracted, precipitated, and resuspended in 10 µl of DNA buffer as previously described. Finally, the NdeI-linearized Klenow blunt-ended pGEM-3Z was dephosphorylated using calf intestine alkaline phosphatase (CIAP) (purchased from Promega Corp., Madison, WI) following the instructions obtained from the supplier. The reaction mixture was incubated for 45 minutes at 37°C, and the DNA was then extracted consecutively with phenol/chloroform and ether, ethanol precipitated, and pelleted by centrifugation. Final pellet was redissolved in 10 µl of DNA buffer.

### C. Blunt End Ligation to Produce pCD385

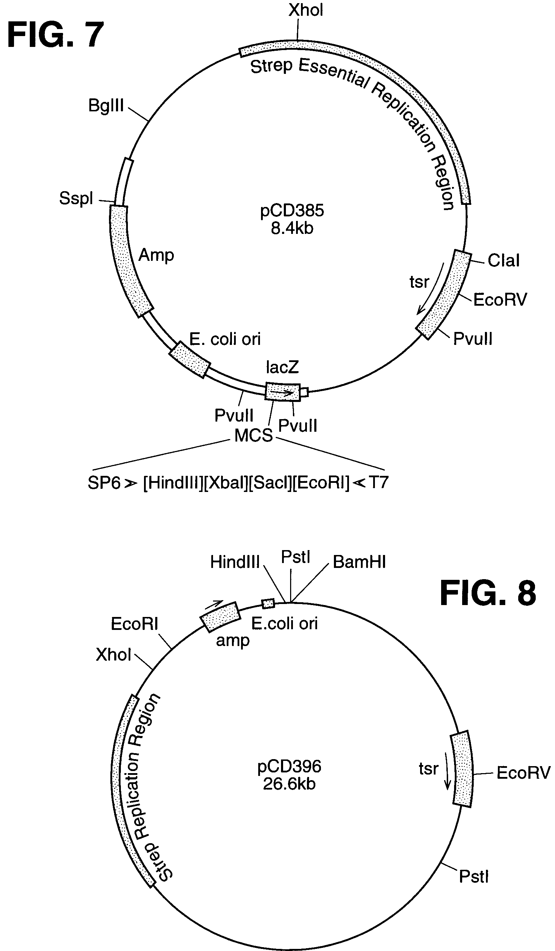

About 8 µl of the SacI-linearized, T4 DNA polymerase-treated pMT660 (see Example 5A), and about 1 µl of the NdeI-linearized, Klenow-treated, CIAP-dephosphorylated pGEM-3Z were incubated overnight with 1 unit of ligase (New England BioLabs, Inc., Beverly, MA) under similar conditions to those described in Example 3C. Next morning, the ligation mixture was used to transform competent E. coli strain DH5-alpha as previously discussed. Selected transformant was designated as strain CD385. The identified transformant was then used for subsequent production and isolation of shuttle vector pCD385. A restriction map of shuttle vector pCD385 is presented in Figure 7.

### EXAMPLE 6

### Construction of Shuttle Vector pCD396

### A. BamHI/EcoRI Double Digestion of pIJ922

Purified plasmid pIJ922 was prepared from a S. lividans transformant as described in Example 1. Following this, approximately 3 µg of the plasmid pIJ922 in 10 µl of DNA buffer were incubated with 10 units each of the restriction enzymes BamHI and EcoRI (purchased from Boehringer Mannheim Biochemicals) in the assay buffer specified as "B" by the supplier, at 37°C for 2 hours, in a total reaction volume of 30 µl. Following restriction, the DNA was extracted twice with an equal volume of phenol- chloroform, twice with an equal volume of ether, and finally the DNA was precipitated by adding 2 volumes of absolute ethanol. Precipitated DNA was recovered by centrifugation and dried under vacuum. The BamHI/EcoRI-treated DNA was then dissolved in 12 µl of DNA buffer.

### B. BamHI/EcoRI Double Digestion and Dephosphorylation of Plasmid pGEM-3Zf(+)

pGEM-3Zf(+) is a 3.2 kb E. coli vector purchased from Promega Corporation, Madison, WI. Purified plasmid pGEM-3Zf(+) was prepared from an E. coli transformant as described in Example 1. Following this, approximately 1 µg of the plasmid pGEM-3Zf(+) in 10 µl of DNA buffer was incubated with 10 units each of the restriction enzymes BamHI and EcoRI (purchased from Boehringer Mannheim Biochemicals) in the assay buffer specified as "B" by the supplier, at 37°C for 2 hours, in a total reaction volume of 30 µl. Then, the BamHI/EcoRI-treated pGEM-3Zf(+) was dephosphorylated using calf intestine alkaline phosphatase (CIAP) (purchased from Promega Corp., Madison, WI) as described above. The DNA was then extracted consecutively with phenol/chloroform and ether, ethanol precipitated, and pelleted by centrifugation. Final pellet was redissolved in 12 µl of DNA buffer.

### C. Ligation to Produce pCD396

About 10 µl of the BamHI/EcoRI-treated pIJ922 (see Example 6A), and about 3 µl of the BamHI/EcoRI-treated, CIAP-dephosphorylated pGEM-3Zf(+) were incubated overnight with 1 unit of ligase (New England BioLabs, Inc., Beverly, MA) under the conditions specified by the supplier at 15°C in a total reaction volume of 20 µl. Next morning, the ligation mixture was used to transform competent E. coli strain DH5-alpha as previously discussed. Selected transformant was designated as strain CD396. The identified transformant was then used for subsequent production and isolation of shuttle vector pCD396. A restriction map of shuttle vector pCD396 is presented in Figure 8.

### EXAMPLE 7

### Transformation of Streptomyces lividans with Shuttle Vectors and Large Scale Preparation of Plasmid Vectors from S. lividans Transformants

S. lividans strain TK64 was used in these experiments. The media for growing and protoplasting S. lividans, and the preparation of protoplasts and transformation were performed as described by D. A. Hopwood et al., Genetic Manipulation of Streptomyces - a laboratory manual, 1985, The John Innes Foundation, Norwich, U. K. In addition, the latter reference has a full description of S. lividans strain TK64. S. lividans protoplasts transformed with shuttle vector pCD262, pCD385, pCD396, or pCD500 were selected for thiostrepton resistance. Plasmid DNAs were prepared from selected transformants as described in Example 1. Vector pCD396 was found to transform S. lividans with an efficiency similar to its parent actinomycetes replicon, namely pIJ922. Vectors pCD262, 385, and 500 were found to transform S. lividans with an efficiency similar to its parent actinomycetes replicon, namely pMT660.

### EXAMPLE 8

### Transformation of Streptomyces avermitilis with Shuttle Vectors.

### A. Preparation of Viable Protoplasts of Streptomyces avermitilis

S. avermitilis strain ATCC 31272 was used throughout. Mycelial cultures were grown in Tripticase Soy Broth (TSB) to a turbidity of 2 to 9 at 600 nm. The culture was homogenized 10 times with a glass tissue grinder. The homogenized mycelia were diluted two-fold in TSB and 20 ml was added to a sterile polypropylene centrifuge tube. An ultrasonic probe (Bronwill Biosonik III) was submerged to a depth of 1 to 2 cm into the liquid, and the sample sonicated at 50% intensity (half maximum wattage) for 10 seconds. Sonication dispersed the mycelial masses into single or double cellular units which produced rapid exponential growth when subcultured. Sonicated mycelial preparations were diluted to a final concentration of 40% glycerol, pipetted into vials, and frozen at -85°C. The media for growing and protoplasting S. avermitilis, and the preparation of protoplasts and transformation were performed as described by D. A. Hopwood et al., Genetic Manipulation of Streptomyces - a laboratory manual, 1985, The John Innes Foundation, Norwich, U. K., following modifications as described by MacNeil, D. J., and Klapko, L. M., J. Industrial Microbiol., 1987, 2, 209-218. S. avermitilis protoplasts transformed with shuttle vector pCD262, pCD385, pCD396, or pCD500 were selected for thiostrepton resistance. Plasmid DNA used to transform S. avermitilis protoplasts were prepared from S. lividans transformants as described in Example 1. Vector pCD396 was found to transform S. avermitilis with an efficiency similar to its parent actinomycetes replicon, namely plJ922. Vectors pCD262, 385, and 500 were found to transform S. avermitilis with an efficiency similar to its parent actinomycetes replicon, namely pMT660.

### EXAMPLE 9

### Transformation of Escherichia coli with Shuttle Vectors

Competent E. coli strain DH5-alpha cells (purchased from Life Technologies, Inc., Gaithersburg, MD) were transformed with purified plasmid DNA by the method recommended by the suppliers. Recovered ampicillin-resistant transformants were grown, plasmid isolated and analyzed following standard procedures (Maniatis et al., Molecular Cloning - a laboratory manual, Cold Spring Harbor Laboratory, 1989).

## Claims

1. A recombinant DNA shuttle vector having an origin of replication that mediates a moderate to high copy number in streptomycetes cells comprising:
(a) the origin of replication-containing SacI restriction fragment approximately 5.7 kilobases in size of plasmid pMT660;
(b) a DNA segment containing at least one antibiotic marker that confers resistance when transformed into a sensitive Streptomyces host cell;
(c) the origin of replication-containing SacI restriction fragment approximately 2.7 kilobases in size of plasmid pGEM-3Z;
(d) a DNA segment containing at least one antibiotic marker that confers resistance when transformed into a sensitive E. coli host cell;
(e) four convenient cloning sites: BgIII, EcoRI, HindIII, and XbaI; and
(f) said vector capable of replicating efficiently in both a wide variety of streptomycetes and E. coli hosts.

2. The shuttle vector of claim 1 which is a plasmid.

3. The plasmid of claim 2 designated pCD262.

4. A transformed host cell comprising a recombinant DNA shuttle vector of claim 1 selected from the group consisting of streptomycetes and E. coli.

5. The transformed host cell of claim 4 selected from the group consisting of Streptomyces avermitilis; Streptomyces coelicolor, Streptomyces hygroscopicus, and Streptomyces lividans.

6. The transformed host cell of claim 5 in which the recombinant DNA cloning shuttle vector is plasmid pCD262.

7. The transformed host cell of claim 4 which is E. coli.

8. The transformed host cell of claim 7 in which the recombinant DNA cloning shuttle vector is plasmid pCD262.

9. A recombinant DNA shuttle vector having an origin of replication that mediates a moderate to high copy number in streptomycetes cells comprising:
(a) the origin of replication-containing SacI restriction fragment approximately 5.7 kilobases in size of plasmid pMT660;
(b) a DNA segment containing at least one antibiotic marker that confers resistance when transformed into a sensitive Streptomyces host cell;
(c) the origin of replication-containing SacI restriction fragment approximately 2.7 kilobases in size of plasmid pGEM-3Z;
(d) a DNA segment containing at least one antibiotic marker that confers resistance when transformed into a sensitive E. coli host cell;
(e) five convenient cloning sites: EcoRI, HindIII, SacI, SphI, and PstI;
(f) said vector capable of replicating efficiently in both a wide variety of streptomycetes and E. coli hosts.

10. The shuttle vector of claim 9 which is plasmid.

11. The plasmid of claim 10 designated pCD500.

12. A transformed host cell comprising a recombinant DNA shuttle vector of claim 9 selected from the group consisting of streptomycetes and E. coli.

13. The transformed host cell of claim 12 selected from the group consisting of Streptomyces avermitilis; Streptomyces coelicolor, Streptomyces hygroscopicus, and Streptomyces lividans.

14. The transformed host cell of claim 13 in which the recombinant DNA cloning shuttle vector is plasmid pCD500.

15. The transformed host cell of claim 12 which is E. coli.

16. The transformed host cell of claim 15 in which the recombinant DNA cloning shuttle vector is plasmid pCD500.

17. A recombinant DNA shuttle vector having an origin of replication that mediates a moderate to high copy number in streptomycetes cells comprising:
(a) the blunt-ended origin of replication-containing SacI restriction fragment approximately 5.7 kilobases in size of plasmid pMT660;
(b) a DNA segment containing at least one antibiotic marker that confers resistance when transformed into a sensitive Streptomyces host cell;
(c) the blunt-ended origin of replication-containing Ndel restriction fragment approximately 2.7 kilobases in size of plasmid pGEM-3Z;
(d) a DNA segment containing at least one antibiotic marker that confers resistance when transformed into a sensitive E. coli host cell;
(e) six convenient cloning sites: BgIII, EcoRI, HindIII, SacI, SspI, and XbaI;
(f) four of the cloning sites (EcoRI, HindIII, SacI, and XbaI) located within the alpha-peptide coding region of the beta-galactosidase gene, allowing recombinant clones to be identified by color screening in E. coli;
(g) SP6 and T7 RNA polymerase promoters flanking the cloning sites allowing the synthesis of cRNA transcripts; and
(h) said vector capable of replicating efficiently in both a wide variety of streptomycetes and E. coli hosts.

18. The shuttle vector of claim 17 which is a plasmid.

19. The plasmid of claim 18 designated pCD385.

20. A transformed host cell comprising a recombinant DNA shuttle vector of claim 17 selected from the group consisting of streptomycetes and E. coli.

21. The transformed host cell of claim 20 selected from the group consisting of Streptomyces avermitilis; Streptomyces coelicolor, Streptomyces hygroscopicus, and Streptomyces lividans.

22. The transformed host cell of claim 21 in which the recombinant DNA cloning shuttle vector is plasmid pCD385.

23. The transformed host cell of claim 20 which is E. coli.

24. The transformed host cell of claim 20 in which the recombinant DNA cloning shuttle vector is plasmid pCD385.

25. A recombinant DNA shuttle vector having an origin of replication that mediates a low copy number in streptomycetes cells comprising:
(a) the origin of replication-containing BamHI/EcoRI restriction fragment approximately 23.4 kilobases in size of plasmid plJ922;
(b) a DNA segment containing at least one antibiotic marker that confers resistance when transformed into a sensitive Streptomyces host cell;
(c) the origin of replication-containing BamHI/EcoRI restriction fragment approximately 3.2 kilobases in size of plasmid pGEM-3Zf(+);
(d) a DNA segment containing at least one antibiotic marker that confers resistance when transformed into a sensitive E. coli host cell,
(e) four convenient cloning sites: BamHI, EcoRI, HindIII, and XhoI; and
(f) said vector capable of replicating efficiently in both a wide variety of streptomycetes and E. coli hosts.

26. The shuttle vector of claim 25 which is a plasmid.

27. The plasmid of claim 26 designated pCD396.

28. A transformed host cell comprising a recombinant DNA shuttle vector of claim 25 selected from the group consisting of streptomycetes and E. coli.

29. The transformed host cell of claim 28 selected from the group consisting of Streptomyces avermitilis; Streptomyces coelicolor, Streptomyces hygroscopicus, and Streptomyces lividans.

30. The transformed host cell of claim 29 in which the recombinant DNA cloning shuttle vector is plasmid pCD396.

31. The transformed host cell of claim 28 which is E. coli.

32. The transformed host cell of claim 31 in which the recombinant DNA cloning shuttle vector is plasmid pCD396.
